# EUROPÄISCHE PATENTSCHRIFT

(11) **EP 2 072 026 B1**
(45) Veröffentlichungstag und Bekanntmachung des Hinweises auf die Patenterteilung: **24.03.2010**
(21) Anmeldenummer: 08018714.9
(22) Anmeldetag: 25.10.2008
(51) Int. Cl.: A61F 2/18

(54) **Modulare Mittelohr-Totalprothese**
Modular middle ear total prosthetic
Prothèse totale de l'oreille moyenne modulaire

(30) Priorität: 21.12.2007 DE 102007062151
(43) Veröffentlichungstag der Anmeldung: 24.06.2009
(73) Patentinhaber: Heinz Kurz GmbH Medizintechnik, 72144 Dusslingen (DE)
(72) Erfinder: Steinhardt, Uwe, 72145 Hirrlingen (DE); Schmid, Georg, Dr., 72072 Tübingen (DE); Kurz, Heinz, 72144 Dusslingen (DE); Heckmann, Walter, 72108 Rottenburg (DE)
(74) Vertreter: Kohler Schmid Möbus

(56) Entgegenhaltungen:
- WO-A-99/42060
- DE-U1- 20 014 659
- DE-U1- 29 904 770
- DE-U1-202007 012 217
- US-B1- 6 168 625

## Beschreibung

Die Erfindung betrifft eine Mittelohrprothese zur totalen Rekonstruktion der menschlichen Gehörknöchelchenkette mit einem schaftförmigen Prothesenkörper, an dessen einem Ende ein erstes Koppelelement vorgesehen ist, das entweder als Kopfplatte zur mechanischen Verbindung der Prothese mit dem Trommelfell oder als Clip zum Ankoppeln der Prothese am Hammergriff ausgeführt ist, und an dessen anderem Ende ein zweites Koppelelement zur mechanischen Verbindung der Prothese mit der Steigbügelfußplatte vorgesehen ist, das ein starr mit dem Prothesenkörper verbundenes Aufnahmeteil sowie ein Einschiebeteil umfasst, welches ein koaxial zur Längsachse des schaftförmigen Prothesenkörpers in das Aufnahmeteil einschiebbares Steckelement sowie einen starr mit dem Steckelement verbundenen Schuh aufweist, der im implantierten Zustand der Prothese auf der Steigbügelfußplatte anliegt.

In der Ausgestaltung mit einer Kopfplatte zur Anlage am Trommelfell ist eine derartige Totalprothese bekannt aus der EP 1 181 907 B1.

Auch die DE 299 04 770 U1 beschreibt Mittelohrprothesen, die zwischen Trommelfell und Steigbügelfußplatte angebracht werden sollen. Jedoch umfasst bei diesen bekannten Prothesen das zweite Koppelelement zur mechanischen Verbindung mit der Steigbügelfußplatte weder ein Aufnahmeteil noch ein Einschiebeteil. Vielmehr ist ein Aufnahmeteil am Ende eines ersten Schaftabschnitts kurz hinter dem als Kopfplatte zur Anlage am Trommelfell ausgebildeten ersten Koppelelement vorgesehen. In dieses Aufnahmeteil kann ein aus einem daran anschließenden zweiten Schaftabschnitt ragendes Steckelement eingeführt werden, um den ersten Schaftabschnitt mit dem zweiten Schaftabschnitt zu verbinden, an dessen entgegengesetztem anderen Ende als zweites Koppelelement ein Schuh zur Anlage an der Steigbügelfußplatte einstückig angebracht ist.

In der DE 20 2004 012 148 U1 ist ein Mittelohrimplantat beschrieben, welches ein erstes Koppelelement zum Hammergriff oder Trommelfell einerseits und als zweites Koppelelement einen Prothesensockel mit einer Prothesenschuhplatte zur Verbindung mit der Steigbügelfußplatte andererseits aufweist. Das Verbindungsteil zwischen den beiden Koppelelementen, das bei den meisten anderen Mittelohrprothesen nach den Stand der Technik von einem Stabförmigen Prothesenschaft gebildet wird, soll hier "konisch-mikrogeschlitzt" ausgeführt sein, so dass zwei längliche, abschnittsweise konische und abschnittsweise parallele Schenkel vom ersten Koppelelement weg mit Abstand voneinander in Richtung auf das zweite Koppelelement verlaufen. In den Spalt zwischen den beiden Schenkeln soll eine Kugel eingesteckt werden, die starr mit der von der Steigbügelfußplatte weg gerichteten Oberseite der Prothesenschuhplatte verbunden ist. Eine über den konischen Teil der beiden Schenkel gestreifte Hülse soll die beiden Schenkel sowie die Kugel dazwischen zusammenpressen. Damit soll eine gelenkige Ankopplung des Prothesensockels an das Verbindungsteil zwischen den beiden Koppelelementen erreicht werden.

Eine Ausbildung des ersten Koppelelements einer Mittelohrprothese als Clip zum Ankoppeln am Hammergriff ist per se bereits bekannt, etwa aus der DE 203 10 609 U1. Die dort beschriebenen Prothesen weisen allerdings kein als Schuh zur Auflage auf der Steigbügelfußplatte ausgebildete zweites Koppelelement auf, sondern entweder einen Kolben zur direkten Verbindung mit dem Innenohr oder einen zweiten Clip.

Die DE 20 2007 012 217 U1 wiederum beschreibt Mittelohrprothesen mit einem als Schuh ausgebildeten zweiten Koppelelement zur mechanischen Verbindung mit der Steigbügelfußplatte. Auch kann das dort beschriebene erste Koppelelement als Clip zur Befestigung der Prothese an einem anderen Glied der Gehörknöchelchenkette ausgebildet sein, jedoch gerade nicht am Hammergriff, sondern beispielsweise am Ambossfortsatz. Außerdem ist das als Schuh ausgebildete zweite Koppelelement nicht starr mit einem in ein Aufnahmeteil einschiebbaren Steckelement verbunden.

Das menschliche Mittelohr mit seinen Gehörknöchelchen hat die Aufgabe, die über den äußeren Gehörgang auf das Trommelfell auftreffenden Schallwellen auf das mit Flüssigkeit gefüllte Innenohr zu übertragen. Die drei Gehörknöchelchen sind der am Trommelfell befestigte Hammer (lat. malleus), der Steigbügel (lat. stapes), der über seine Fußplatte (lat. basis stapedis) mit dem Innenohr verbunden ist, und der Amboss (lat. incus), der sich zwischen dem Hammer und dem Steigbügel befindet und mit diesen gelenkig verbunden ist. Beispielsweise die Otosklerose ist eine Erkrankung des menschlichen Felsenbeins (= Knochen, in dem das gesamte Ohr sitzt), bei der es durch entzündungsähnliche Knochenumbauprozesse zu einer Fixierung des normalerweise locker schwingenden Steigbügels kommen kann. Dadurch wird das Schallsignal nicht oder nur unvollkommen über die Gehörknöchekhenkette auf das Innenohr übertragen, was zur Schwerhörigkeit führt.

Mittelohrprothesen dienen der Verbesserung der Schall-übertragung bei unterschiedlichen pathologischen Befunden. Sie werden verwendet, um bei ganz oder teilweise fehlenden oder geschädigten Gehörknöchelchen des menschlichen Mittelohrs den Schall vom Trommelfell zum Innenohr zu übertragen. Drei besonders häufig verwendete Arten von Mittelohrprothesen sind die Steigbügel-Prothesen, die Partial-Prothesen und die Total-Prothesen. Steigbügel-Prothesen (=Stapes-Prothesen) werden am Amboss fixiert und ragen über einen Stempel (=Piston) ins Innenohr. Partial-Prothesen liegen meist mit einer Kopfplatte am Trommelfell an und stellen eine Verbindung zum Steigbügelköpfchen her. Total-Prothesen verbinden das Trommelfell mit der SteigbügelFußplatte. Die vorliegende Erfindung betrifft ausschließlich Total-Prothesen.

Ein Hauptproblem, das bei jeder Rekonstruktion der menschlichen Gehörknöchelchenkette auftaucht, ist die Auswahl der richtigen Prothesenlänge. Anatomisch bedingt, variieren die jeweils erforderlichen Längen in einem Spektrum von mehreren Millimetern. Daher muss beim operativen Einsetzen einer Mittelohrprothese entweder eine ausreichend große Auswahl von Prothesen unterschiedlicher axialer Länge bereitgehalten werden oder die verwendeten Gehörknöchelchenprothesen müssen während der Operation ausgehend von einer maximalen oder minimalen Ausgangslänge auf die erforderliche axiale Endlänge gebracht werden können.

Ein weiteres gravierendes Problem insbesondere der Total-Prothesen liegt darin, dass Ossikel-Rekonstruktionen bei fehlendem Amboss und gleichzeitig fehlendem Steigbügeloberbau per se immer das hohe Risiko einer Instabilität oder Dislokation der Prothese in sich tragen. Hinzu kommen unterschiedliche, patientenindividuelle anatomische Gegebenheiten, die präoperativ in der Regel nicht eindeutig feststellbar sind, und die nicht nur die erforderliche axiale Länge der Prothese betreffen, sondern auch zahlreiche andere geometrische Parameter. Diese machen es für den Operateur schwer und aufwändig, die jeweils optimale Verbindung zwischen Prothese und Steigbügelfußplatte dauerhaft zu erzielen.

Aufgabe der vorliegenden Erfindung ist es demgegenüber, eine gattungsgemäße Mittelohrprothese der eingangs beschriebenen Art mit möglichst einfachen technischen Mitteln dahin gehend zu verbessern, dass einerseits eine gewünschte, definierte Länge der Prothese auch schon vor ihrem Einsetzen zwischen den beiden Befestigungspunkten leicht hergestellt werden kann, wobei diese Länge auch nach Abschluss der Operation fix beibehalten wird, und dass andererseits während der Operation auch auf die jeweils vorgefundene individuelle Situation des Steigbügels des Patienten (oder dessen Reste) ad hoc reagiert und eine optimale geometrische Anbindung der Prothese an die Steigbügelfußplatte erreicht werden kann, ohne dass eine große Vielzahl verschieden ausgestalteter Mittelohrprothesen bereitgehalten werden muss.

Erfindungsgemäß wird diese Aufgabe auf ebenso überraschend einfache wie wirkungsvolle Art und Weise dadurch gelöst, dass das Aufnahmeteil an seinem dem Prothesenkörper abgewandten Ende eine als länglicher Hohlraum ausgebildete Aufnahmeöffnung mit zylindrischer Bohrung aufweist, die sich in axialer Richtung des Prothesenkörpers erstreckt und das Steckelement des Einschiebeteils im montierten Zustand vollumfänglich umgibt, dass der lichte Durchmesser des länglichen Hohlraums größer ist als die maximale Querausdehnung des Steckelements quer zur Längsachse des schaftförmigen Prothesenkörpers, dass der längliche Hohlraum in Richtung vom Einschiebeteil weg einseitig geschlossen ist und im montierten Zustand als Endanschlag für das Steckelement wirkt, und dass das zweite Koppelelement sich in axialer Richtung der Prothese maximal ein Drittel der axialen Länge des Prothesenkörpers erstreckt.

Auf diese Weise kann je nach der - erst während der Operation vorgefundenen - individuellen Situation der Steigbügelumgebung des Patienten durch einfaches Einstecken des Steckelements eines Einschiebeteiles geeigneter axialer Länge und mit geometrisch optimalem Schuh in den länglichen Hohlraum des Aufnahmeteiles der Prothese eine genau auf den Patienten abgestimmte optimale Implantationssituation hergestellt werden.

Besonders hilfreich ist hierbei, dass die individuelle Anpassung nicht eine Bevorratung größere Sortimente von Prothesen unterschiedlicher Längen und deren Bereithaltung während jeder Operation erfordert. Vielmehr kann stets eine einheitliche "Standard-Prothese mit Kopfplatte, Schaft und Aufnahmeteil verwendet werden, welche - wie oben beschrieben - durch einfaches Einstecken eines aus einem Sortiment ausgesuchten Einschiebeteiles optimal individualisiert wird.

Dies ermöglicht unaufwändig und kostengünstig sowohl eine nahezu beliebige Längenvariabilität als auch eine optimale Geometrieanpassung der Mittelohrprothese "in situ" bzw. ad hoc intraoperativ. Die Einstellung der jeweils gewünschten individuellen Prothesenlänge und Geometrie und damit deren Handhabung sind besonders einfach.

Nachträgliche postoperative unerwünschte Längen- und/oder Lageänderungen der Prothese werden durch die erfindungsgemäß ermöglichte optimale Anpassung sicher vermieden.

Ganz besonders bevorzugt ist eine Ausführungsform der erfindungsgemäßen Mittelohrprothese, bei der sich das zweite Koppelelement in axialer Richtung der Prothese maximal ein Viertel der axialen Länge des Prothesenkörpers erstreckt, so dass insbesondere ein Abknicken der Prothese bei der Montage des Steigbügelseitigen Endabschnitts durch Ankoppeln des Einschiebeteils im Aufnahmeteil sicher vermieden wird.

Bei einer Klasse von Ausführungsformen der Erfindung ist das Einschiebeteil auf wählbaren diskreten relativen koaxialen Positionen längs einer axialen Strecke mit dem Aufnahmeteil mittels einer äußeren Einwirkung auf das Einschiebeteil und/oder auf das Aufnahmeteil aktiv verklemmbar, so dass sich damit nahezu jede gewünschte Prothesenlänge unterhalb der vom Grundmuster der Prothese vorgegebenen Maximallänge individuell genau einstellen lässt.

Bei einfachen Weiterbildungen dieser Ausführungsformen kann das Verklemmen durch äußere mechanische Krafteinwirkung, insbesondere mittels Einwirkung einer Crimpzange in radialer Richtung auf das Aufnahmeteil, bewirkt werden.

Bei eleganteren, allerdings in der Herstellung dafür auch etwas aufwändigeren Weiterbildungen ist vorgesehen, dass das Einschiebeteil durch Wärmeeintrag auf die Mittelohrprothese von außen, insbesondere mittels Erwärmung der Mittelohrprothese auf Körpertemperatur, mit dem Aufnahmeteil aktiv verklemmbar ist.

Ganz besonders vorteilhaft sind Varianten dieser Weiterbildungen, bei denen das Einschiebeteil und/oder das Aufnahmeteil ganz oder teilweise aus einem Material mit Formgedächtnis (=memory effect) insbesondere aus Nitinol oder einem Polymer hergestellt ist. Die Verwendung solcher Materialien ist auf dem Gebiet der Mittelohrprothesen an sich bekannt, erweist sich aber gerade im Zusammenhang mit der vorliegenden Erfindung als besonders wirkungsvoll.

Aus herstellungstechnischen Gründen sind Ausführungsformen der erfindungsgemäßen Mittelohrprothese zu bevorzugen, bei denen das Steckelement eine durchgehend zylindrische Form aufweist.

Eine geometrisch und ergonomisch günstige Ausführungsform der erfindungsgemäßen Mittelohrprothese zeichnet sich dadurch aus, dass das Steckelement eine an seinem im montierten Zustand dem Aufnahmeteil zugewandten axialen Ende angeordnete Verdickung aufweist. Damit lässt sich auch eine besonders gute Ankoppelung des Einschiebeteils im Aufnahmeteil zu erzielen.

In der Praxis bewähren sich Weiterbildungen dieser Ausführungsform, bei denen die Verdickung des Steckelements eine ellipsoide, insbesondere eine rotationsellipsoide, vorzugsweise eine kugelige Form aufweist. Diese Geometrien bereiten auch fertigungstechnisch keine großen Probleme.

Auf diese Weise kann das zweite Koppelelement problemlos als Kugelgelenk ausgebildet werden, was die weiter unten beschriebenen Vorteile hat.

Besonders einfach zu fertigen sind Aufnahmeteile, die in ihrem Querschnitt senkrecht zu seiner Längsachse eine zylindrische Außenkontur aufweisen. Möglich sind aber nicht nur kreiszylindrische Formen, sondern etwa auch Aufnahmeteile mit rechteckigem Querschnitt.

Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Bei einer steifen Verbindung zwischen Kopfplatte und Schaft kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse post-operative Mobilität aufweist, so dass sich die Kopfplatte postoperativ selbstständig der Position des Trommelfells angleichen kann. Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Mittelohrprothesen nicht starr ausgebildet sind, sondern eine gewisse Flexibilität oder Variabilität aufweisen.

In der Regel wird bei der erfindungsgemäßen Gehörknöchelchen-Prothese das Verbindungselement zwischen den beiden Befestigungselementen als länglicher Schaft gestaltet sein, wie dies an sich aus dem Stand der Technik wohlbekannt ist. Um die oben erörterte erhöhte Flexibilität bzw. Variabilität der Prothese zu erreichen - wie beispielsweise ausführlich in der EP 1 181 907 B1 beschrieben - kann bei einer besonders bevorzugten Ausführungsform der Erfindung am oder im mindestens ein Gelenk, insbesondere ein Kugelgelenk vorgesehen sein. Vorteilhaft im Hinblick auf eine besonders hohe postoperative Beweglichkeit der Prothese sind Weiterbildungen, bei denen der Prothesenkörper eine Vielzahl von aneinander angrenzenden weiteren Drehelementen, vorzugsweise eine axial verlaufende Kugelgelenkkette aufweist.

Die erfindungsgemäße Mittelohrprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Prothese oder Teile davon, insbesondere eines der Befestigungselemente, aus einem Material mit Formgedächtnis (=memory effect) oder superelastischen Eigenschaften, vorzugsweise aus Nitinol oder einem Polymer hergestellt sind, was per se beispielsweise aus der WO 02/069850 A1 oder der US 6,554,861 B2 bekannt ist.

Alternativ oder ergänzend können bei weiteren Ausführungsformen Teile der erfindungsgemäßen Mittelohrprothese aus einem Keramikmaterial hergestellt sein.

Möglich sind aber auch Ausführungsformen der Erfindung, bei denen die gesamte Prothese oder Teile davon aus biokompatiblen Kunststoffen, insbesondere Silikon, Polytetrafluorethylen (PTFE) oder Faserverbundwerkstoffen, insbesondere Kohlefasern oder einem Thermoplast hergestellt sind. Mit diesen Materialien können post-operative Abstoßungsreaktionen in den meisten Fällen ebenfalls verhindert werden.

Neben der postoperativen Positionsverschiebung ergibt sich nach der Implantation von Mittelohrprothesen auch noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Mittelohrprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Mittelohrprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen. Die Kopfplatte der erfindungsgemäßen Mittelohrprothese sollte grundsätzlich eine wachstumsfördernde Beschichtung aufweisen.

Besonders bevorzugt ist eine Ausführungsform der erfindungsgemäßen Mittelohrprothese, bei der die Massenverteilung der einzelnen Teile der Prothese in Abhängigkeit von einem gewünschten, vorgebbaren Frequenzgang der Schallleitung im Mittelohr berechnet ist. Damit lässt sich ohne großen zusätzlichen technischen Aufwand gewissermaßen ein Tuning der Schallfortpflanzungseigenschaften mittels einer individuellen ausgestatteten Mittelohrprothese erreichen.

Ein solcher Tuning Effekt kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist. Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

In den Rahmen der vorliegenden Erfindung fällt auch eine Gruppe von Mittelohrprothesen der oben beschriebenen erfindungsgemäßen Art mit einem mit einem Einschiebeteil zum Einschieben in das Aufnahmeteil, bei dem der starr mit dem Steckelement verbundene Schuh, der im implantierten Zustand der Prothese auf der Steigbügelfußplatte anliegt, platten- oder stempelförmig aufgebaut ist.

Eine bevorzugte Klasse von Ausführungsformen zeichnet sich dadurch aus, dass der im implantierten Zustand der Mittelohrprothese auf der Steigbügelfußplatte anliegende Endabschnitt des Schuhes eine in Richtung auf das Steckelement konkav gewölbte Fläche aufweist, was in der Regel den besten Halt der Prothese auf der Steigbügelfußplatte gewährleistet.

Für individuell andere Geometrien der vorgefundenen Steigbügelreste beim Patienten kann der Einsatz einer alternativen Klasse von Ausführungsformen günstig sein, bei welchen der im implantierten Zustand der Mittelohrprothese auf der Steigbügelfußplatte anliegende Endabschnitt des Schuhes flach ausgebildet ist und eine großflächige Auflage gewährleistet.

Eine weitere Klasse von Einschiebeteilen für spezielle Einsatzzwecke zeichnet sich dadurch aus, dass der im implantierten Zustand der Mittelohrprothese auf der Steigbügelfußplatte anliegende Endabschnitt des Schuhes eine in Richtung vom Steckelement weg konvex gewölbte Fläche aufweist und damit eine Punktauflage ermöglicht.

Bei bevorzugten Weiterbildungen dieser Klasse von Einschiebeteilen weist der Endabschnitt des Schuhes eine rotationsellipsoide, vorzugsweise eine kugelige Form auf, die relativ einfach zu fertigen ist.

Zur besseren Befestigung des Einschiebeteils am Steigbügel kann bei Weiterbildungen der Endabschnitt des Schuhes eine im implantierten Zustand der Mittelohrprothese gegen die Steigbügelfußplatte gerichtete Spitze aufweisen, was ein Einbohren des Schuhes in die Steigbügelfußplatte ermöglicht und eine größere Haftwirkung durch erhöhte Friktion bewirkt.

Als vorteilhaft erweisen sich oftmals Weiterbildungen, bei denen der Schuh an seinem Außenumfang radial nach außen ragende Stabilisatorflügel aufweist, die u.a. eine Zentrierung der Anordnung in der ovalen Nische der Steigbügelfußplatte ermöglichen.

Alternativ kann bei Weiterbildungen des erfindungsgemäßen Einschiebeteils der Schuh an seinem Außenumfang radial nach innen verlaufende Aussparungen aufweisen, in die individuell beim Patienten vorhandene Knochenreste des Steigbügels eingreifen und so einen festen Halt der Prothese ermöglichen.

Bei anderen individuellen Befunden kann auch eine Geometrie des Einschiebeteils günstig sein, bei welcher der Schuh einen geringeren maximalen Außenumfang als das Steckelement aufweist. Damit lässt sich auf einfache Weise eine Reduktion des Durchmessers und mithin der wirksamen Auflagefläche des Schuhes auf der Steigbügelfußplatte erreichen.

Allgemein ist es aus hygienischen Gründen dringend anzuraten, dass das Einschiebeteil bis zum Einschieben in das Aufnahmeteil in einer sterilen, vorzugsweise luftdichten Verpackung gelagert ist.

Weiterhin fällt in den Rahmen der vorliegenden Erfindung auch eine Mittelohrprothese, bei der ein Sortiment von erfindungsgemäß ausgestalteten Einschiebeteilen vorgesehen ist, die in unterschiedlichen geometrischen Ausformungen vorliegen, aber derart ausgebildet sind, dass die Mittelohrprothese nach dem Einschieben des jeweiligen Steckelements eines Einschiebeteils bis zum Endanschlag in den länglichen Hohlraum des Aufnahmeteils jeweils die gleiche axiale Gesamtlänge aufweist.

In diesem Zusammenhang ist eine Ausführungsform vorteilhaft, die sich dadurch auszeichnet, dass die Gesamtlänge der Mittelohrprothese nach dem Einschieben des Steckelements bis zum Endanschlag in den länglichen Hohlraum des Aufnahmeteils zwischen 0,3mm und 0,8mm, vorzugsweise 0,5mm beträgt. Besonders bevorzugt werden die Einschiebeteile so gestaltet sein, dass sie zwar unterschiedliche Geometrien aufweisen, aber in ihrem im Aufnahmeteil eingeschobenen Zustand zur jeweils gleichen axialen Länge der Mittelohrprothese führen.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:
- Fig. 1a: eine schematische räumliche Darstellung einer ersten Ausführungsform der erfindungsgemäßen Mittelohrprothese mit einer Trommelfell-Kopfplatte als erstem Befestigungselement;
- Fig. 1b: einen schematischen Schnitt durch die Ausführungsform nach Fig. 1a in ihrer postoperativen Einbausituation im Mittelohr;
- Fig. 2: eine Ausführungsform mit einem Clip als erstem Befestigungselement sowie mit einem Kugelgelenk im Prothesenkörper; und
- Fign. 3a-f: verschiedene, geometrisch und funktionell unterschiedliche Ausführungsformen des erfindungsgemäßen Einschiebeteils.

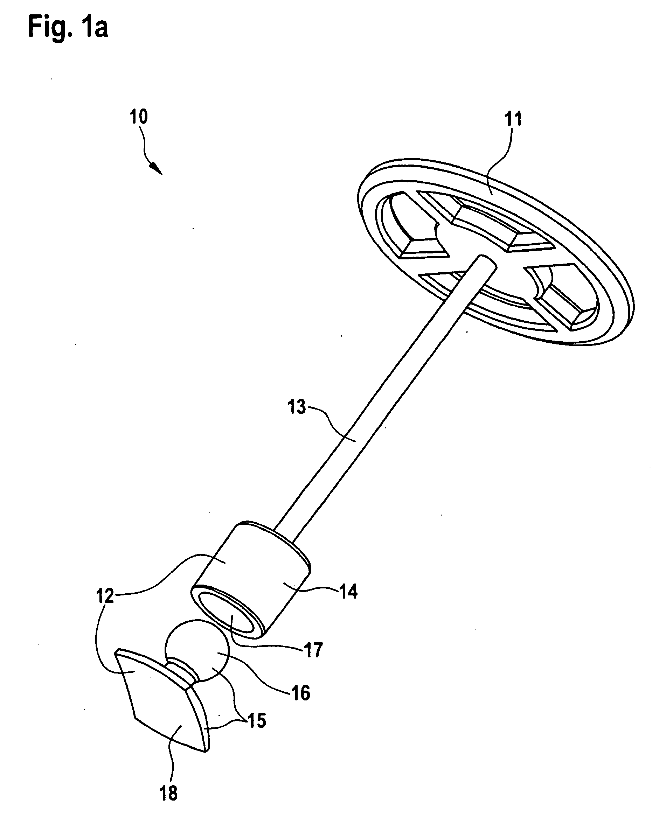

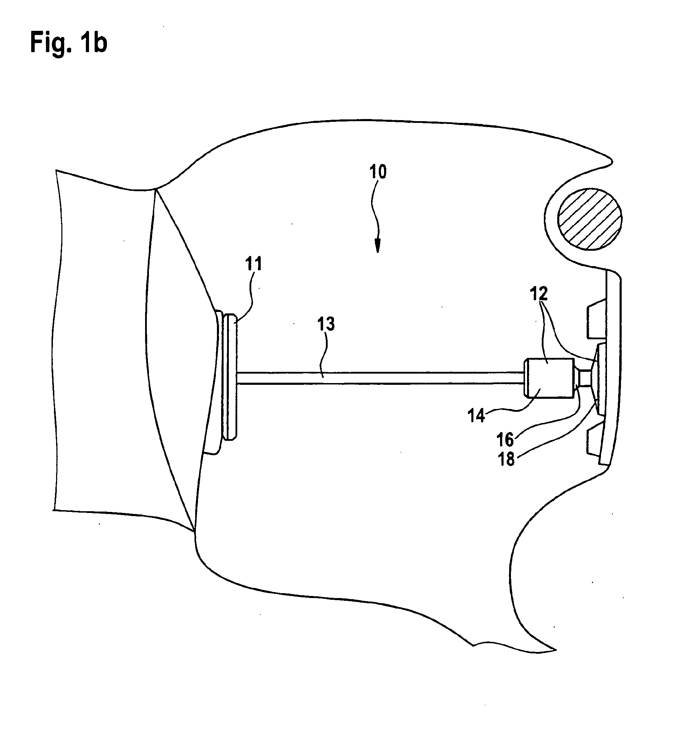

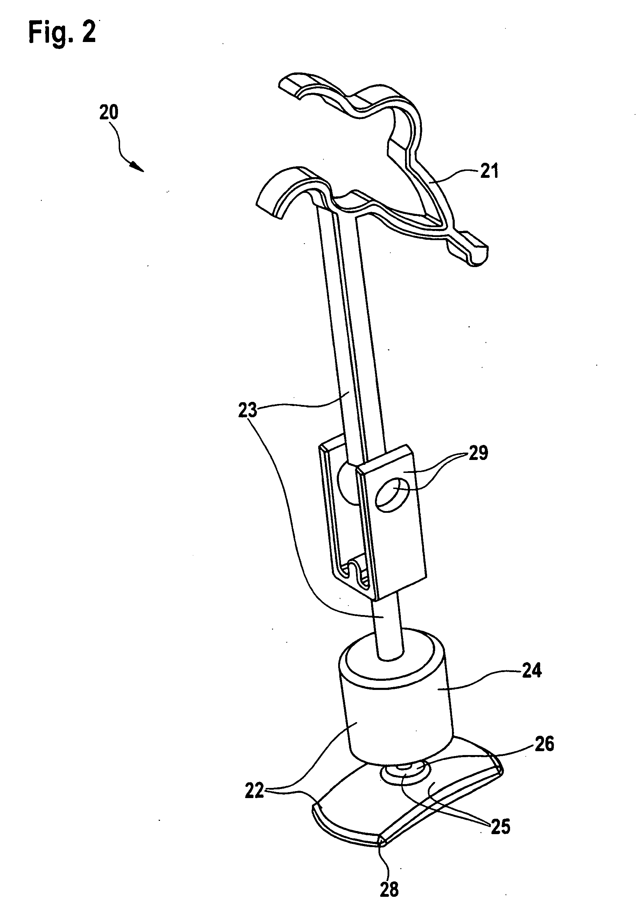

Die in den Figuren 1a, 1b und 2 schematisch dargestellten, im Detail unterschiedlich gestalteten Ausführungsformen der erfindungsgemäßen **Mittelohrprothese 10; 20** weisen an ihrem einen Ende jeweils ein **erstes Befestigungselement 11; 21** auf.

Dabei ist das Befestigungselement 11 in den Figuren 1a und 1b als Kopfplatte zur Anlage der Prothese am Trommelfell ausgebildet, das Befestigungselement 21 in Fig. 2 weist die Form eines Clips zur mechanischen Verbindung am Hammergriff auf.

Am anderen Ende der Mittelohrprothese 10; 20 sitzt jeweils ein **zweites Befestigungselement 12; 22** zum mechanischen Ankoppeln der Prothese an der Steigbügelfußplatte. Dazwischen ist ein die beiden Befestigungselemente 11; 21 bzw. 12; 22 Schall leitend miteinander verbindender, als länglicher Schaft ausgebildeter **Prothesenkörper 13; 23** angeordnet.

Das zweite Befestigungselement 12; 22 umfasst jeweils ein als **Aufnahmeteil 14; 24** ausgebildetes, starr mit dem Prothesenkörper 13; 23 verbundenes erstes Teilstück sowie ein als **Einschiebeteil 15; 25** ausgebildetes zweites Teilstück, welches ein koaxial zur Längsachse des Prothesenkörpers 13; 23 in eine Aufnahmeöffnung des Aufnahmeteils 14; 24 einschiebbares **Steckelement 16; 26** sowie einen starr mit dem Steckelement 16; 26 verbundenen **Schuh 18; 28** aufweist, der im implantierten Zustand der Prothese 10; 20 auf der Steigbügelfußplatte anliegt

Erfindungsgemäß weist das Aufnahmeteil 14; 24 an seinem dem Prothesenkörper 13; 23 abgewandten Ende jeweils eine als **länglicher Hohlraum 17** ausgebildete Aufnahmeöffnung mit zylindrischer Bohrung auf, die sich in axialer Richtung des Prothesenkörpers 13; 23 erstreckt und das Steckelement 16; 26 des Einschiebeteils 15; 25 im montierten Zustand vollumfänglich umgibt. Der lichte Durchmesser des länglichen Hohlraums 17 ist größer als die maximale Querausdehnung des Steckelements 16; 26 quer zur Längsachse des schaftförmigen Prothesenkörpers 13; 23, der längliche Hohlraum 17 ist in Richtung vom Einschiebeteil 15; 25 weg einseitig geschlossen und wirkt im montierten Zustand als Endanschlag für das Steckelement 16; 26. Das zweite Koppelelement 12; 22 erstreckt sich in axialer Richtung der Prothese maximal ein Drittel, vorzugsweise maximal ein Viertel der axialen Länge des Prothesenkörpers 13; 23.

In der Ausführungsform nach Fig.2 ist in den Prothesenkörper 23 ein **Kugelgelenk 29** integriert, um eine gewisse postoperative Flexibilität der Mittelohrprothese 20 zwischen ihren Verbindungsstellen zu erreichen.

Die Massenverteilung der einzelnen Teile der erfindungsgemäßen Mittelohrprothese 10; 20 kann in Abhängigkeit von einem gewünschten, vorgebbaren Frequenzgang der Schallleitung im Mittelohr derart berechnet sein, dass ein individuelles Tuning der SchallleitungsEigenschaften ermöglicht wird.

Zumindest abschnittsweise kann eine biologisch aktive Beschichtung, insbesondere eine wachstumshemmende und/oder eine wachstumsfördernde und /oder eine antibakteriell wirkende Beschichtung der Mittelohrprothese 10; 20 vorgesehen sein.

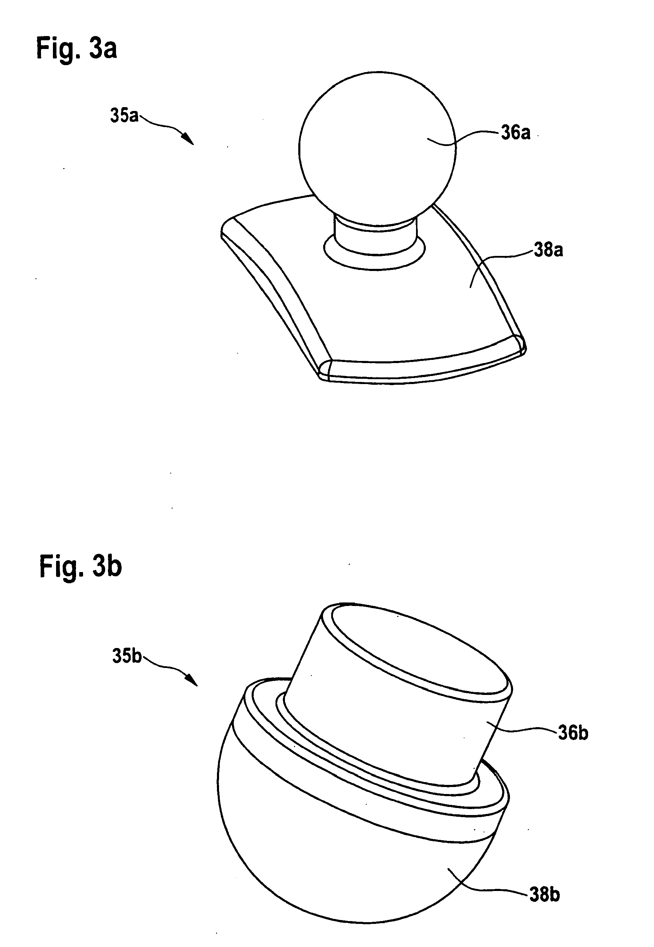

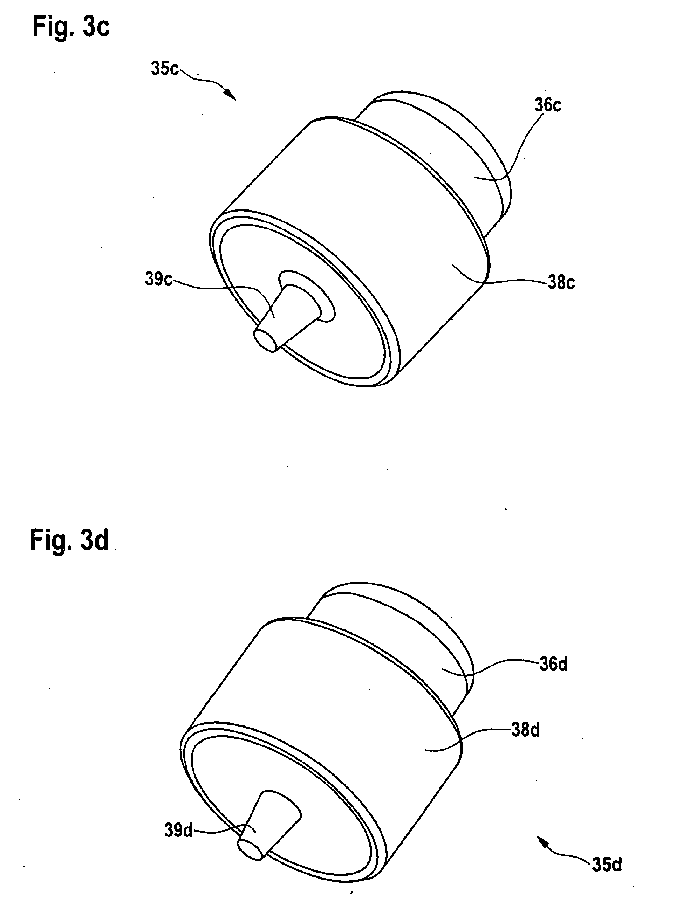

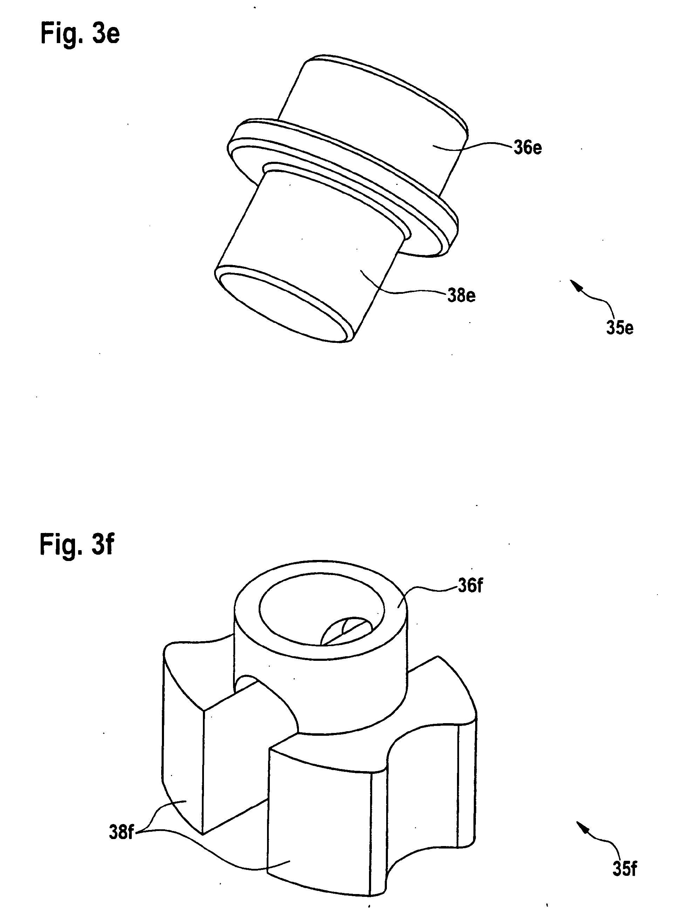

Bei den Ausführungsformen nach den Figuren 3a bis 3f weist das Einschiebeteil 35a; 35b; 35c; 35d; 35e; 35f jeweils verschiedene geometrische Gestaltungen auf, die für unterschiedliche, beim Patienten individuell vorgefundene Situationen im Bereich der Steigbügelfußplatte ad hoc eine optimale Anpassung der Mittelohrprothese ermöglichen, wie weiter oben erläutert wurde:

Das **Einschiebeteil 35a** weist ein kugelförmig verdicktes **Steckelement 36a** sowie einen konkav gewölbten **Schuh 38a** auf.

Das Einschiebeteil 35b umfasst ein zylindrisches **Steckelement 36b** sowie einen konvex in kugeliger Form nach außen gewölbten **Schuh 38b.**

Die **Einschiebeteile 35c; 35d** weisen jeweils ein i.w. zylindrisches **Steckelement 36c; 36d** sowie einen flachen **Schuh 38c; 38d** auf, der auf seinem Endabschnitt jeweils eine im implantierten Zustand der Mittelohrprothese gegen die Steigbügelfußplatte gerichtete **Spitze 39c; 39d** trägt, wobei der Endabschnitt flach, konkav oder auch konvex gewölbt gestaltet sein kann. Die Spitze 39c in Fig. 3c ist in ihrem Ansatzbereich am Endabschnitt des Schuhes 38c zusätzlich mit einem ringförmigen Wulst umgeben, der eine großflächige Auflage des Endabschnitts auf der Steigbügelfußplatte verhindert.

Beim **Einschiebeteil 35e** weist der **Schuh 38e** einen geringeren maximalen Außenumfang als das **Steckelement 36e** auf.

Das **Einschiebeteil 35f** schließlich umfasst ein hohlzylindrisches **Steckelement 36f** sowie einen **Schuh 38f**, der an seinem Außenumfang radial nach innen verlaufende Aussparungen aufweist.

Außer den in den Figuren der Zeichnung dargestellten Ausführungsformen sind auch noch viele andere geometrische Ausgestaltungen des erfindungsgemäßen Einschiebeteils möglich, beispielsweise die oben beschriebene Variante, bei der der Schuh an seinem Außenumfang radial nach außen ragende Stabilisatorflügel aufweist. Damit können praktisch alle denkbaren individuellen Situationen im Bereich der Steigbügelfußplatte eines Patienten abgedeckt werden.

## Patentansprüche

1. Mittelohrprothese (10; 20) zur totalen Rekonstruktion der menschlichen Gehörknöchelchenkette mit einem schaftförmigen Prothesenkörper (13; 23), an dessen einem Ende ein erstes Koppelelement (11; 21) vorgesehen ist, das entweder als Kopfplatte zur mechanischen Verbindung der Prothese mit dem Trommelfell oder als Clip zum Ankoppeln der Prothese am Hammergriff ausgeführt ist, und an dessen anderem Ende ein zweites Koppelelement (12; 22) zur mechanischen Verbindung der Prothese mit der Steigbügelfußplatte vorgesehen ist, das ein starr mit dem Prothesenkörper (13; 23) verbundenes Aufnahmeteil (14; 24) sowie ein Einschiebeteil (15; 25; 35a; 35b; 35c; 35d; 35e; 35f) umfasst, welches ein koaxial zur Längsachse des schaftförmigen Prothesenkörpers (13; 23) in das Aufnahmeteil (14; 24) einschiebbares Steckelement (16; 26; 36a; 36b; 36c; 36d; 36e; 36f) sowie einen starr mit dem Steckelement (16; 26; 36a; 36b; 36c; 36d; 36e; 36f) verbundenen Schuh (18; 28; 38a; 38b; 38c; 38d; 38e; 38f) aufweist, der im implantierten Zustand der Prothese auf der Steigbügelfußplatte anliegt, **dadurch gekennzeichnet, dass** das Aufnahmeteil (14; 24) an seinem dem Prothesenkörper (13; 23) abgewandten Ende eine als länglicher Hohlraum (17) ausgebildete Aufnahmeöffnung mit zylindrischer Bohrung aufweist, die sich in axialer Richtung des Prothesenkörpers (13; 23) erstreckt und das Steckelement (16; 26; 36a; 36b; 36c; 36d; 36e; 36f) des Einschiebeteils (15; 25; 35a; 35b; 35c; 35d; 35e; 35f) im montierten Zustand vollumfänglich umgibt, dass der lichte Durchmesser des länglichen Hohlraums (17) größer ist als die maximale Querausdehnung des Steckelements (16; 26; 36a; 36b; 36c; 36d; 36e; 36f) quer zur Längsachse des schaftförmigen Prothesenkörpers (13; 23), dass der längliche Hohlraum (17) in Richtung vom Einschiebeteil (15; 25; 35a; 35b; 35c; 35d; 35e; 35f) weg einseitig geschlossen ist und im montierten Zustand als Endanschlag für das Steckelement (16; 26; 36a; 36b; 36c; 36d; 36e; 36f) wirkt, und dass das zweite Koppelelement (12; 22) sich in axialer Richtung der Prothese maximal ein Drittel der axialen Länge des Prothesenkörpers (13; 23) erstreckt.

2. Mittelohrprothese nach Anspruch 1, **dadurch gekennzeichnet, dass** das Einschiebeteil (15; 25; 35a; 35b; 35c; 35d; 35e; 35f) auf wählbaren diskreten relativen koaxialen Positionen längs einer axialen Strecke mit dem Aufnahmeteil (14; 24) mittels einer äußeren Einwirkung auf das Einschiebeteil (15; 25; 35a; 35b; 35c; 35d; 35e; 35f) und/oder auf das Aufnahmeteil (14; 24) aktiv verklemmbar ist.

3. Mittelohrprothese nach Anspruch 2 **dadurch gekennzeichnet, dass** das Einschiebeteil (15; 25; 35a; 35b; 35c; 35d; 35e; 35f) durch äußere mechanische Krafteinwirkung, insbesondere mittels Einwirkung einer Crimpzange in radialer Richtung auf das Aufnahmeteil (14; 24), mit dem Aufnahmeteil (14; 24) aktiv verklemmbar ist.

4. Mittelohrprothese nach Anspruch 2, **dadurch gekennzeichnet, dass** das Einschiebeteil (15; 25; 35a; 35b; 35c; 35d; 35e; 35f) durch Wärmeeintrag auf die Mittelohrprothese (10; 20) von außen, insbesondere mittels Erwärmung der Prothese auf Körpertemperatur, mit dem Aufnahmeteil (14; 24) aktiv verklemmbar ist.

5. Mittelohrprothese nach Anspruch 4, **dadurch gekennzeichnet, dass** das Einschiebeteil (15; 25; 35a; 35b; 35c; 35d; 35e; 35f) und/oder das Aufnahmeteil (14; 24) ganz oder teilweise aus einem Material mit Formgedächtnis (=memory effect), insbesondere aus Nitinol oder einem Polymer hergestellt ist.

6. Mittelohrprothese nach einem der Ansprüche 1 bis 5, **dadurch gekennzeichnet, dass** das Steckelement (36b; 36c; 36d; 36e; 36f) eine durchgehend zylindrische Form aufweist.

7. Mittelohrprothese nach einem der Ansprüche 1 bis 5, **dadurch gekennzeichnet, dass** das Steckelement (16; 26; 36a) eine an seinem im montierten Zustand dem Aufnahmeteil (14; 24) zugewandten axialen Ende angeordnete Verdickung mit einer ellipsoiden, insbesondere einer rotationsellipsoiden, vorzugsweise einer kugeligen Form aufweist.

8. Mittelohrprothese nach einem der vorhergehenden Ansprüche, **dadurch gekennzeichnet, dass** das Aufnahmeteil (14; 24) in seinem Querschnitt senkrecht zu seiner Längsachse eine zylindrische Außenkontur aufweist.

9. Mittelohrprothese nach einem der vorhergehenden Ansprüche, **dadurch gekennzeichnet, dass** der Prothesenkörper (23) mindestens ein Gelenk, insbesondere ein Kugelgelenk (29), vorzugsweise eine axial verlaufende Kugelgelenkkette aufweist.

10. Mittelohrprothese nach einem der vorhergehenden Ansprüche, **dadurch gekennzeichnet, dass** der starr mit dem Steckelement (16; 26; 36a; 36b; 36c; 36d; 36e; 36f) verbundene Schuh (18; 28; 38a; 38b; 38c; 38d; 38e; 38f), der im implantierten Zustand der Prothese auf der Steigbügelfußplatte anliegt, platten- oder stempelförmig aufgebaut ist.

11. Mittelohrprothese nach einem Anspruch 10, **dadurch gekennzeichnet, dass** der im implantierten Zustand der Mittelohrprothese (10; 20) auf der Steigbügelfußplatte anliegende Endabschnitt des Schuhes (18; 28; 38a; 38b; 38c; 38d; 38e; 38f) eine in Richtung auf das Steckelement (16; 26; 36a) konkav gewölbte Fläche aufweist und/oder flach ausgebildet ist und/oder eine in Richtung vom Steckelement (36b) weg konvex gewölbte Fläche aufweist.

12. Mittelohrprothese nach Anspruch 11, **dadurch gekennzeichnet, dass** der Endabschnitt des Schuhes (38b) eine rotationsellipsoide, vorzugsweise eine kugelige Form aufweist.

13. Mittelohrprothese nach einem der Ansprüche 11 oder 12, **dadurch gekennzeichnet, dass** der Endabschnitt des Schuhes (38c; 38d) eine im implantierten Zustand der Mittelohrprothese gegen die Steigbügelfußplatte gerichtete Spitze (39c; 39d) aufweist.

14. Mittelohrprothese nach einem der vorhergehenden Ansprüche, **dadurch gekennzeichnet, dass** der starr mit dem Steckelement (16; 26; 36a; 36b; 36c; 36d; 36e; 36f) verbundene Schuh (18; 28; 38a; 38b; 38c; 38d; 38e; 38f) an seinem Außenumfang radial nach außen ragende Stabilisatorflügel und/oder radial nach innen verlaufende Aussparungen aufweist und/oder dass der Schuh (38e) einen geringeren maximalen Außenumfang als das Steckelement (36e) aufweist.

15. Mittelohrprothese nach einem der vorhergehenden Ansprüche, **dadurch gekennzeichnet, dass** ein Sortiment von Einschiebeteilen (15; 25; 35a; 35b; 35c; 35d; 35e; 35f) vorgesehen ist, bei welchem die Einschiebeteile (15; 25; 35a; 35b; 35c; 35d; 35e; 35f) in unterschiedlichen geometrischen Ausformungen vorliegen, aber derart ausgebildet sind, dass die Mittelohrprothese (10; 20) nach dem Einschieben des jeweiligen Steckelements (16; 26; 36a; 36b; 36c; 36d; 36e; 36f) eines Einschiebeteils (15; 25; 35a; 35b; 35c; 35d; 35e; 35f) bis zum Endanschlag in den länglichen Hohlraum (17) des Aufnahmeteils (14; 24) jeweils die gleiche axiale Gesamtlänge aufweist.

## Claims

1. Middle ear prosthesis (10; 20) for total reconstruction of the ossicular chain in humans, having a shaft shaped prosthesis body (13; 23) which, at one end, has a first coupling element (11; 21) realized either as a head plate for mechanical connection of the prosthesis to the ear drum or as a clip for coupling the prosthesis to the handle of the hammer, and which, at the other end, for mechanical connection of the prosthesis to the foot plate of the stirrup, has a second coupling element (12; 22) that comprises a receiving part (14; 24) rigidly connected to the prosthesis body (13; 23) and also an insertion part (15; 25; 35a; 35b; 35c; 35d; 35e; 35f) that has a plug element (16; 26; 36a; 36b; 36c; 36d; 36e; 36f) which can be inserted into the receiving part (14; 24) coaxially with respect to the longitudinal axis of the shaft shaped prosthesis body (13; 23) and also, rigidly connected to the plug element (16; 26; 36a; 36b; 36c; 36d; 36e; 36f), a shoe (18; 28; 38a; 38b; 38c; 38d; 38e; 38f) which in the implanted state of the prosthesis abuts the foot plate of the stirrup, **characterised in that** the receiving part (14; 24), at its end remote from the prosthesis body (13; 23), has a receiving opening realized as an elongate cavity (17) with a cylindrical bore which extends in the axial direction of the prosthesis body (13; 23) and, in the assembled state, fully surrounds the plug element (16; 26; 36a; 36b; 36c; 36d; 36e; 36f) of the insertion part (15; 25; 35a; 35b; 35c; 35d; 35e; 35f); and **in that** the inside diameter of the elongate cavity (17) is greater than the largest transverse extent of the plug element (16; 26; 36a; 36b; 36c; 36d; 36e; 36f) transversely with respect to the longitudinal axis of the shaft shaped prosthesis body (13; 23); and **in that** the elongate cavity (17) is closed off at one end in the direction away from the insertion part (15; 25; 35a; 35b; 35c; 35d; 35e; 35f) and in the assembled state acts as an end abutment for the plug element (16; 26; 36a; 36b; 36c; 36d; 36e; 36f); and **in that** the second coupling element (12; 22) extends in the axial direction of the prosthesis by a maximum of one third of the axial length of the prosthesis body (13; 23).

2. Middle ear prosthesis according to claim 1, **characterised in that** the insertion part (15; 25; 35a; 35b; 35c; 35d; 35e; 35f) can be actively clamped with the receiving part (14; 24 at selectable discrete relative coaxial positions along an axial length by acting externally upon the insertion part (15; 25; 35a; 35b; 35c; 35d; 35e; 35f) and/or upon the receiving part (14; 24).

3. Middle ear prosthesis according to claim 2, **characterised in that** the insertion part (15; 25; 35a; 35b; 35c; 35d; 35e; 35f) can be actively clamped with the receiving part (14; 24) by acting upon the receiving part (14; 24) with external mechanical force, especially by acting in the radial direction with crimping pliers.

4. Middle ear prosthesis according to claim 2, **characterised in that** the insertion part (15; 25; 35a; 35b; 35c; 35d; 35e; 35f) can be actively clamped with the middle ear prosthesis (10; 20) by external application of heat, especially by heating the prosthesis to body temperature.

5. Middle ear prosthesis according to claim 4, **characterised in that** the insertion part (15; 25; 35a; 35b; 35c; 35d; 35e; 35f) and/or the receiving part (14; 24) is made wholly or partly of a material that has shape memory (= memory effect), especially of Nitinol or of a polymer.

6. Middle ear prosthesis according to one of claims 1 to 5, **characterised in that** the plug element (36b; 36c; 36d; 36e; 36f) is of continuously cylindrical shape.

7. Middle ear prosthesis according to one of claims 1 to 5, **characterised in that** the plug element (16; 26; 36a), has a thickening arranged at its axial end which in the assembled state faces the receiving part (14; 24), which is ellipsloidal in shape, especially an ellipsoid of revolution, preferably of spherical shape.

8. Middle ear prosthesis according to one of the preceding claims, **characterised in that** the receiving part (14; 24) has a cylindrical outer contour in its cross section perpendicularly with respect to its longitudinal axis.

9. Middle ear prosthesis according to one of the preceding claims, **characterised in that** the prosthesis body (23) has at least one joint, especially a ball joint (29), preferably an axially extending ball joint chain.

10. Middle ear prosthesis according to one of the preceding claims, **characterised in that** the shoe (18; 28; 38a; 38b; 38c; 38d; 38e; 38f) which is rigidly connected to the plug element (16; 26; 36a; 36b; 36c; 36d; 36e; 36f) and which in the implanted state of the prosthesis abuts the foot plate of the stirrup, is made plate shaped or punch shaped.

11. Middle ear prosthesis according to claim 10, **characterised in that** the end section of the shoe (18; 28; 38a; 38b; 38c; 38d; 38e; 38f) which in the implanted state of the middle ear prosthesis (10; 20) abuts the foot plate of the stirrup, has a surface that is concavely curved in the direction towards the plug element (16; 26; 36a) and/or is made flat and/or has a surface that is convexly curved in the direction away from the plug element (36b).

12. Middle ear prosthesis according to claim 11, **characterised in that** the end section of the shoe (38b) has a shape that is an ellipsoid of revolution, preferably a spherical shape.

13. Middle ear prosthesis according to one of claims 11 or 12, **characterised in that** the end section of the shoe (38c; 38d) has a tip (39c; 39d) which in the implanted state of the middle ear prosthesis is directed towards the foot plate of the stirrup.

14. Middle ear prosthesis according to one of the preceding claims, **characterised in that** the shoe (18; 28; 38a; 38b; 38c; 38d; 38e; 38f) which is rigidly connected to the plug element (16; 26; 36a; 36b; 36c; 36d; 36e; 36f) has at its outer circumference radially outwardly projecting stabiliser wings and/or radially inwardly extending recesses and/or **in that** the shoe (38e) has a smaller maximum outer circumference than the plug element (36e).

15. Middle ear prosthesis according to one of the preceding claims, **characterised in that** an assortment of insertion parts (15; 25; 35a; 35b; 35c; 35d; 35e; 35f) is provided in which the insertion parts (15; 25; 35a; 35b; 35c; 35d; 35e; 35f) are present in different geometrical shapes but are formed such that the middle ear prosthesis (10; 20), after insertion of the respective plug element (16; 26; 36a; 36b; 36c; 36d; 36e; 36f) of an insertion part (15; 25; 35a; 35b; 35c; 35d; 35e; 35f) in each case has the same total axial length up to the end abutment of the elongate cavity (17) of the receiving part (14; 24).

## Revendications

1. Prothèse d'oreille moyenne (10 ; 20) destinée à la reconstruction totale de la chaîne des osselets de l'oreille humaine avec un corps de prothèse (13 ; 23) en forme de tige, à l'une des extrémités duquel il est prévu un premier élément de couplage (11 ; 21), qui se présente sous la forme d'une plaque de tête pour raccorder mécaniquement la prothèse à la membrane tympanique ou sous la forme d'une agrafe pour coupler la prothèse au manche de marteau, et à l'autre extrémité duquel il est prévu un second élément de couplage (12 ; 22) pour raccorder mécaniquement la prothèse à la plaque de base de l'étrier, qui comprend une partie de réception (14 ; 24) raccordée de manière rigide au corps de prothèse (13 ; 23), ainsi qu'une partie d'insertion (15 ; 25 ; 35a ; 35b ; 35c ; 35d ; 35e ; 35f), qui présente un élément (16 ; 26 ; 36a ; 36b ; 36c ; 36d ; 36e ; 36f) enfichable coaxialement à l'axe longitudinal du corps de prothèse (13 ; 23) en forme de tige dans la partie de réception (14 ; 24), ainsi qu'un sabot (18 ; 28 ; 38a ; 38b ; 38c ; 38d ; 38e ; 38f) raccordé de manière rigide à l'élément enfichable (16 ; 26 ; 36a ; 36b ; 36c ; 36d ; 36e ; 36f), sabot qui s'applique à l'état implanté de la prothèse sur la plaque de base de l'étrier, **caractérisée en ce que** la partie de réception (14 ; 24) présente, à son extrémité opposée au corps de prothèse (13 ; 23), une ouverture réceptrice conformée en espace creux oblong (17) avec un perçage cylindrique, qui s'étend dans la direction axiale du corps de prothèse (13 ; 23) et qui entoure entièrement l'élément enfichable (16 ; 26 ; 36a ; 36b ; 36c ; 36d ; 36e ; 36f) de la partie d'insertion (15 ; 25 ; 35a ; 35b ; 35c ; 35d ; 35e ; 35f) à l'état monté, **en ce que** le diamètre intérieur de l'espace creux oblong (17) est supérieur à l'extension transversale maximale de l'élément enfichable (16 ; 26 ; 36a ; 36b ; 36c ; 36d ; 36e ; 36f) transversalement à l'axe longitudinal du corps de prothèse (13 ; 23) en forme de tige, **en ce que** l'espace creux oblong (17) est fermé d'un côté dans la direction opposée à la partie d'insertion (15 ; 25 ; 35a ; 35b ; 35c ; 35d ; 35e ; 35f) et agit à l'état monté comme butée d'extrémité pour l'élément enfichable (16 ; 26 ; 36a ; 36b ; 36c ; 36d ; 36e ; 36f) et **en ce que** le second élément de couplage (12 ; 22) s'étend dans la direction axiale de la prothèse au maximum sur un tiers de la longueur axiale du corps de prothèse (13 ; 23).

2. Prothèse d'oreille moyenne selon la revendication 1, **caractérisée en ce que** la partie d'insertion (15 ; 25 ; 35a ; 35b ; 35c ; 35d ; 35e ; 35f) peut être serrée activement sur des positions coaxiales relatives discrètes sélectionnables le long d'une distance axiale avec la partie de réception (14 ; 24) au moyen d'une action externe sur la partie d'insertion (15 ; 25 ; 35a ; 35b ; 35c ; 35d ; 35e ; 35f) et/ou sur la partie de réception (14 ; 24).

3. Prothèse d'oreille moyenne selon la revendication 2, **caractérisée en ce que** la partie d'insertion (15 ; 25 ; 35a ; 35b ; 35c ; 35d ; 35e ; 35f) peut être serrée activement avec la partie de réception (14 ; 24) par l'action d'une force mécanique externe, en particulier par l'action d'une pince à sertir dans la direction radiale sur la partie de réception (14 ; 24) .

4. Prothèse d'oreille moyenne selon la revendication 2, **caractérisée en ce que** la partie d'insertion (15 ; 25 ; 35a ; 35b ; 35c ; 35d ; 35e ; 35f) peut être serrée activement avec la partie de réception (14 ; 24) par apport de chaleur sur la prothèse d'oreille moyenne (10 ; 20) de l'extérieur, en particulier par chauffage de la prothèse à la température du corps.

5. Prothèse d'oreille moyenne selon la revendication 4, **caractérisée en ce que** l'on fabrique la partie d'insertion (15 ; 25 ; 35a ; 35b ; 35c ; 35d ; 35e ; 35f) et/ou la partie de réception (14 ; 24), en totalité ou en partie, en matériau à mémoire de forme (= memory effect), en particulier en nitinol ou en un polymère.

6. Prothèse d'oreille moyenne selon l'une quelconque des revendications 1 à 5, **caractérisée en ce que** l'élément enfichable (36b ; 36c ; 36d ; 36e ; 36f) présente une forme cylindrique de bout en bout.

7. Prothèse d'oreille moyenne selon l'une quelconque des revendications 1 à 5, **caractérisée en ce que** l'élément enfichable (16 ; 26 ; 36a) présente un épaississement aménagé à son extrémité axiale tournée à l'état monté vers la partie de réception (14 ; 24) avec une forme ellipsoïdale, en particulier sphéroïde, de préférence sphérique.

8. Prothèse d'oreille moyenne selon l'une quelconque des revendications précédentes, **caractérisée en ce que** la partie de réception (14 ; 24) présente un contour externe cylindrique dans sa section transversale perpendiculairement à son axe longitudinal.

9. Prothèse d'oreille moyenne selon l'une quelconque des revendications précédentes, **caractérisée en ce que** le corps de prothèse (23) présente au moins une articulation, en particulier une articulation à rotule (29), de préférence une chaîne d'articulations à rotule s'étendant axialement.

10. Prothèse d'oreille moyenne selon l'une quelconque des revendications précédentes, **caractérisée en ce que** le sabot (18 ; 28 ; 38a ; 38b ; 38c ; 38d ; 38e ; 38f) raccordé de manière rigide à l'élément enfichable (16 ; 26 ; 36a ; 36b ; 36c ; 36d ; 36e ; 36f) et qui s'applique à l'état implanté de la prothèse sur la plaque de base de l'étrier, est construit en forme de plaque ou de matrice.

11. Prothèse d'oreille moyenne selon la revendication 10, **caractérisée en ce que** la section terminale du sabot (18 ; 28 ; 38a ; 38b ; 38c ; 38d ; 38e ; 38f) s'appliquant à l'état implanté de la prothèse d'oreille moyenne (10 ; 20) sur la plaque de base de l'étrier présente une surface incurvée de manière concave dans la direction de l'élément enfichable (16 ; 26 ; 36a) et/ou est de conformation plate et/ou présente une surface incurvée de manière convexe dans la direction opposée à l'élément enfichable (36b).

12. Prothèse d'oreille moyenne selon la revendication 11, **caractérisée en ce que** la section terminale du sabot (38b) présente une forme sphéroïde, de préférence sphérique.

13. Prothèse d'oreille moyenne selon l'une quelconque des revendications 11 ou 12, **caractérisée en ce que** la section terminale du sabot (38c ; 38d) présente une pointe (39c ; 39d) dirigée à l'état implanté de la prothèse d'oreille moyenne à l'encontre de la plaque de base de l'étrier.

14. Prothèse d'oreille moyenne selon l'une quelconque des revendications précédentes, **caractérisée en ce que** le sabot (18 ; 28 ; 38a ; 38b ; 38c ; 38d ; 38e ; 38f) raccordé de manière rigide à l'élément enfichable (16 ; 26 ; 36a ; 36b ; 36c ; 36d ; 36e ; 36f) présente des ailes stabilisatrices faisant saillie sur son pourtour externe radialement vers l'extérieur et/ou des évidements s'étendant radialement vers l'intérieur et/ou **en ce que** le sabot (38e) présente un pourtour externe maximal plus petit que celui de l'élément enfichable (36e).

15. Prothèse d'oreille moyenne selon l'une quelconque des revendications précédentes, **caractérisée en ce qu'**il est prévu un assortiment de parties d'insertion (15 ; 25 ; 35a ; 35b ; 35c ; 35d ; 35e ; 35f), dans lequel les parties d'insertion (15 ; 25 ; 35a ; 35b ; 35c ; 35d ; 35e ; 35f) sont disponibles sous différentes formes géométriques, mais conçues de sorte que la prothèse d'oreille moyenne (10 ; 20) présente respectivement la même longueur axiale totale après insertion de l'élément enfichable respectif (16 ; 26 ; 36a ; 36b ; 36c ; 36d ; 36e ; 36f) d'une partie d'insertion (15 ; 25 ; 35a ; 35b ; 35c ; 35d ; 35e ; 35f) jusqu'à la butée d'extrémité dans l'espace creux oblong (17) de la partie de réception (14 ; 24).
